# EUROPÄISCHE PATENTSCHRIFT

(11) **EP 3 801 673 B1**
(45) Veröffentlichungstag und Bekanntmachung des Hinweises auf die Patenterteilung: **16.10.2024**
(21) Anmeldenummer: 19729209.7
(22) Anmeldetag: 30.05.2019
(51) Int. Cl.: A61M 60/13, A61M 60/878, A61M 60/816, A61M 60/237

(54) **MOTORGEHÄUSEMODUL FÜR EIN HERZUNTERSTÜTZUNGSSYSTEM SOWIE HERZUNTERSTÜTZUNGSSYSTEM UND VERFAHREN ZUM MONTIEREN EINES HERZUNTERSTÜTZUNGSSYSTEMS**
MOTOR HOUSING MODULE FOR A HEART SUPPORT SYSTEM, HEART SUPPORT SYSTEM AND METHOD FOR MOUNTING A HEART SUPPORT SYSTEM
MODULE DE CARTER DE MOTEUR DESTINÉ À UN SYSTÈME D'ASSISTANCE CARDIAQUE AINSI QUE SYSTÈME D'ASSISTANCE CARDIAQUE ET PROCÉDÉ DE MONTAGE D'UN SYSTÈME D'ASSISTANCE CARDIAQUE

(30) Priorität: 30.05.2018 DE 102018208539
(43) Veröffentlichungstag der Anmeldung: 14.04.2021
(73) Patentinhaber: Kardion GmbH, 70376 Stuttgart (DE)
(72) Erfinder: KASSEL, Julian, 75181 Pforzheim (DE); MINZENMAY, David, 70569 Stuttgart (DE); SCHLEBUSCH, Thomas, Alexander, 71272 Renningen (DE)
(74) Vertreter: Gauss, Nikolai
(86) Internationale Anmeldenummer: PCT/EP2019/064156
(87) Internationale Veröffentlichungsnummer: WO 2019/229222

(56) Entgegenhaltungen:
- WO-A1-2013/120957
- WO-A1-94/09835
- WO-A1-99/49912

## Beschreibung

Die Erfindung betrifft ein Motorgehäusemodul zum Abdichten eines Motorraums eines Motors eines Herzunterstützungssystems und ein Herzunterstützungssystem sowie ein Verfahren zum Montieren eines Herzunterstützungssystems.

Aus der WO 99/49912 A1 ist ein Herzunterstützungssystem mit einem Motorgehäusemodul mit einem in einem abgedichteten Motorraum angeordneten Motor bekannt. Das Motorgehäusemodul hat einen Durchführungsabschnitt zum Herstellen einer elektrischen Verbindung zwischen dem Herzunterstützungssystem und einem Anschlusskabel zum externen Kontaktieren des Herzunterstützungssystems. Das Motorgehäusemodul weist eine Durchführleitung auf, die in dem Durchführungsabschnitt eingebettet ist und sich durch den Durchführungsabschnitt hindurch erstreckt, wobei die Durchführleitung an den Motor und an das Anschlusskabel anschließbar ist.

Herzunterstützungssysteme, wie beispielsweise ein linksventrikuläres Herzunterstützungssystem, können in eine Herzkammer implantiert werden und integrierte elektronische Komponenten, beispielsweise Sensoren, aufweisen. Die Integration elektronischer Komponenten in das Herzunterstützungssystem erfolgt meist klassisch auf Substraten, z. B. Leiterplatten oder gedruckten Leiterplatten (PCBs, printed circuit boards), aufgebaut und in entsprechend große Kavitäten des Herzunterstützungssystems integriert. Diese Herzunterstützungssysteme können beispielsweise mittels einer Sternotomie implantiert werden. Zudem ist es möglich, kompakter gebaute Herzunterstützungssysteme, beispielsweise auch linksventrikuläre Herzunterstützungssysteme, minimalinvasiv in ein Blutgefäß zu implantieren. Diese kompakter gebauten Herzunterstützungssysteme weisen aufgrund der Anforderungen an die Baugröße bisher keine integrierten elektronischen Komponenten mit implantiert platzierter Verarbeitungselektronik auf.

Die US 9,474,840 B2 beschreibt die Integration eines optischen Drucksensors in die Spitze eines kompakter gebauten Herzunterstützungssystems zum minimalinvasiven Implantieren. Die optische Zuleitung ist mittels einer Glasfaser in einem Kanal aufwendig realisiert. Die gesamte Auswerteelektronik wird durch die Glasfaser abgesetzt in einer extrakorporalen Steuerkonsole platziert. Für vollimplantierte Systeme ist es jedoch erforderlich, auch die Verarbeitungselektronik implantiert zu platzieren.

Aufgabe der Erfindung ist es, ein verbessertes Herzunterstützungssystem bereitzustellen. Insbesondere ist es eine Aufgabe der Erfindung, in einem Herzunterstützungssystem auf kleinem Bauraum sowohl für einen Motor zum Antreiben einer die Herzfunktion unterstützenden Blutpumpe als auch für Sensoren elektrische Anschlussmöglichkeiten zu schaffen.

Diese Aufgabe wird durch ein Motorgehäusemodul mit den in Anspruch 1 angegebenen Merkmalen sowie ein Herzunterstützungssystem nach Anspruch 13 und das in Anspruch 14 angegebene Verfahren zum Montieren eines Herzunterstützungssystems gelöst.

Vorteilhafte Ausführungsformen der Erfindung sind in den abhängigen Ansprüchen angegeben.

Vor diesem Hintergrund werden mit dem hier vorgestellten Ansatz ein Motorgehäusemodul zum Abdichten eines Motorraums eines Motors eines Herzunterstützungssystems, ein Herzunterstützungssystem sowie ein Verfahren zum Montieren eines Herzunterstützungssystems gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Mit diesem Ansatz wird ein Motorgehäusemodul für ein Herzunterstützungssystem vorgestellt. Das Motorgehäusemodul kann den Motorraum des Herzunterstützungssystems fluiddicht abdichten und den Motor des Herzunterstützungssystems mit einem Anschlusskabel verbinden, über das der Motor mit Strom versorgt werden kann. Zudem können mittels dem Motorgehäusemodul Sensorsignale zusammengeführt, verarbeitet und über das Anschlusskabel weitergeleitet werden. Das Motorgehäusemodul und das Herzunterstützungssystem können dabei vorteilhafterweise so kompakt gestaltet sein, das sie beispielsweise für ein linksventrikuläres Herzunterstützungssystem (LVAD, left ventricular assist device) zum minimalinvasiven Implantieren als vollimplantiertes System verwendet werden können. Insbesondere kann das Herzunterstützungssystem so ausgebildet sein, dass es sich mittels Katheter in eine Herzkammer oder die Aorta einführen lässt.

Vorteilhafterweise ist es somit möglich, auch in einem kompakt gebauten Herzunterstützungssystem elektronische Bauelemente zu integrieren.

Es wird ein Motorgehäusemodul zum Abdichten eines Motorraums eines Motors eines Herzunterstützungssystems vorgestellt. Das Motorgehäusemodul weist einen Durchführungsabschnitt, zumindest eine Durchführleitung und zumindest einen Kontaktstift auf. Der Durchführungsabschnitt ist ausgebildet, eine elektrische Verbindung zwischen dem Herzunterstützungssystem und einem Anschlusskabel zum externen Kontaktieren des Herzunterstützungssystems herzustellen. Die zumindest eine Durchführleitung ist in dem Durchführungsabschnitt eingebettet und erstreckt sich durch den Durchführungsabschnitt hindurch. Die Durchführleitung ist an den Motor und an das Anschlusskabel anschließbar. Ein erstes Ende des zumindest einen Kontaktstifts ist in den Durchführungsabschnitt eingebettet und ein zweites Ende ragt auf einer von dem Motorraum abgewandten Seite aus dem Durchführungsabschnitt heraus. Das zweite Ende des Kontaktstifts ist an eine Sensorleitung zu zumindest einem Sensor des Herzunterstützungssystems und an das Anschlusskabel anschließbar.

Das Motorgehäusemodul kann beispielsweise einteilig oder zweiteilig ausgeführt sein. Beispielsweise kann das Motorgehäusemodul Titankomponenten oder Glaskomponenten aufweisen. Das Herzunterstützungssystem kann beispielsweise ein linksventrikuläres Herzunterstützungssystem sein, das eine Herzpumpe mit einem Motor aufweist. Bei dem Motorraum kann es sich beispielsweise um einen Abschnitt des Herzunterstützungssystems handeln, beispielsweise auch um einen Gehäuseabschnitt. Der Motorraum kann mittels des hier vorgestellten Gehäuses vorteilhafterweise hermetisch, also fluiddicht, abgedichtet werden. Das Motorgehäusemodul kann beispielsweise aus einem Material bestehen, das eine Schweißverbindung zwischen dem Motor oder dem Motorraum und dem Motorgehäusemodul ermöglicht, um den Motorraum abzudichten. Der Durchführungsabschnitt zum Herstellen einer elektrischen Verbindung zwischen dem Herzunterstützungssystem und dem Anschlusskabel kann beispielsweise einteilig ausgeführt sein. Alternativ kann der Durchführungsabschnitt beispielsweise einen Frästeil und eine Glaskomponente umfassen, die beispielsweise durch Laserschweißen oder Sintern hermetisch miteinander verbunden sind. Die Durchführleitung und der Kontaktstift können beispielsweise aus einem elektrisch leitfähigen Material, beispielsweise einem Metall wie einer Eisen-Nickel-Kobalt-Legierung mit einem geringen Wärmeausdehnungskoeffizienten oder Edelstahl bestehen. Das Anschlusskabel zum externen Kontaktieren des Herzunterstützungssystems kann beispielsweise eine elektrische Verbindung zu einer weiteren implantierten Komponente herstellen, beispielsweise einer Stromquelle und/oder Steuereinheit des Herzunterstützungssystems. Die Sensorleitung kann beispielsweise eine Gruppe von Leitungen umfassen und ausgebildet sein, um Sensorsignale eines Sensors im Pumpenkopf des Herzunterstützungssystems und/oder Sensorsignale mehrerer Sensoren weiterzuleiten. Die Sensorleitung kann beispielsweise als ein aufgebrachtes flexibles Dünnschichtsubstrat realisiert sein.

Der Durchführungsabschnitt kann gemäß einer Ausführungsform zumindest eine mit einem elektrisch isolierenden Material verfüllte Durchgangsöffnung zum Einbetten der zumindest einen Durchführleitung und zumindest ein mit einem elektrisch isolierenden Material verfülltes Sackloch zum Einbetten des zumindest einen Kontaktstifts aufweisen. Vorteilhafterweise kann der Durchführungsabschnitt somit beispielsweise aus Glas hergestellt sein oder werden, und sowohl die Durchführleitung als auch der Kontaktstift können eingebettet werden. Diese Ausführungsform ermöglicht vorteilhafterweise eine besonders kostensparende Herstellung.

Von Vorteil ist es zudem gemäß einer Ausführungsform, wenn die zumindest eine Durchführleitung und zusätzlich oder alternativ der zumindest eine Kontaktstift zylinderförmig oder kelchförmig ausgeformt sind. Wenn die zumindest eine Durchführleitung und der zumindest eine Kontaktstift zylinderförmig, also als gerade Pins, ausgeführt sind, kann die Anbindung des Anschlusskabels beispielsweise durch direktes Löten, Kleben, Crimpen oder Schweißen der Anschlusskabellitzen an den Pin oder unter Verwendung einer Hülse oder eines Steckers erfolgen. Bei einer kelchförmig oder tulpenförmigen Ausformung der zumindest einen Durchführleitung und zusätzlich oder alternativ des zumindest einen Kontaktstifts kann das Anbinden an das Anschlusskabel beispielsweise durch ein Einführen der Litzen des Anschlusskabels in den Kelch der Durchleitung oder des Kontaktstifts erfolgen, wobei das Fixieren mittels Löten, Kleben, Crimpen oder Schweißen realisiert werden kann. Vorteilhafterweise lassen sich gemäß dieser Ausführungsform verschiedene Anwendungsformen realisieren, was in Bezug auf eine möglichst einfache Bauweise von Vorteil ist. Zudem kann beispielsweise eine zusätzliche mechanische Stabilisierung der Anbindung erfolgen, beispielsweise durch einen Stecker als Teil der Anbindung.

Gemäß einer Ausführungsform kann das Motorgehäusemodul einen Korpus umfassen. Der Korpus kann eine Sensornut zum Aufnehmen zumindest eines elektronischen Bauteils, insbesondere eines Sensors und zusätzlich oder alternativ eines Sensor-Hubs, aufweisen. Vorteilhafterweise kann somit ein Sensor auf dem Korpus des Motorgehäusemoduls platziert werden, was eine kompakte Bauweise ermöglicht. Die elektrische Kontaktierung eines in der Sensornut aufgenommenen elektronischen Bauteils mit dem Durchführungsabschnitt kann beispielsweise mittels eines elektrisch leitfähigen Substrats, beispielsweise eines flexiblen Dünnschichtsubstrats, erfolgen. Die Sensornut kann beispielsweise auch als Mulde oder als Kavität ausgeformt sein. Der Korpus kann beispielsweise ein Frästeil aus Titan sein. Der Korpus kann beispielsweise ausgeformt sein, um den Durchführungsabschnitt zu umschließen. Der Durchführungsabschnitt, der beispielsweise Glas aufweisen kann, kann dann durch Laserschweißen, Sintern oder Umspritzen hermetisch mit dem Frästeil kombiniert werden. Eine Integration des Durchführungsabschnitts in den Korpus kann in Bezug auf die Bauweise vorteilhaft sein, da der Korpus des Motorgehäusemoduls besonders einfach mit einem anderen Abschnitt des Herzunterstützungssystems, beispielsweise dem Motorraum oder dem Motor, verschweißt werden kann.

Wenn das Motorgehäusemodul gemäß einer Ausführungsform eine Sensornut aufweist, kann das Motorgehäusemodul zusätzlich eine Sensorkappe zum Abdecken des zumindest einen in der Sensornut aufgenommenen elektronischen Bauteils aufweisen. Die Sensorkappe kann beispielsweise ein Metall aufweisen und durch Kleben fixiert werden. Vorteilhafterweise kann so ein aufgenommenes elektronisches Bauteil durch die Sensorkappe geschützt werden.

Zudem kann das Motorgehäuse gemäß dieser Ausführungsform einen Sensorleitungsabschnitt der Sensorleitung aufweisen. Der Sensorleitungsabschnitt kann im Bereich der Sensornut einen Sensorträger zum Anschließen des zumindest einen elektronischen Bauteils ausformen. Der Sensorleitungsabschnitt repräsentiert einen Teil der Sensorleitung des Herzunterstützungssystems, die Sensorleitung kann dazu beispielsweise modular ausgeführt sein. Zum Ausformen des Sensorträgers kann sich die Sensorleitung beispielsweise im Bereich des Sensorleitungsabschnitts aufweiten. Vorteilhafterweise ist gemäß dieser Ausführungsform eine Verbindung mit der Sensorleitung und eine Integration eines elektronischen Bauteils, wie beispielsweise eines weiteren Sensors, besonders platzsparend und einfach möglich.

Das elektronische Bauteil kann gemäß einer Ausführungsform einen Sensor-Hub aufweisen. Der Sensor-Hub kann ausgebildet sein, zumindest ein Sensorsignal des zumindest einen Sensors des Herzunterstützungssystems zu verarbeiten. Zusätzlich oder alternativ kann der Sensor-Hub dazu ausgebildet sein, das Sensorsignal über den zumindest einen Kontaktstift an das Anschlusskabel bereitzustellen. Unter dem Sensor-Hub kann beispielsweise ein Gerät verstanden werden, das Netzknoten mehrerer Sensoren beispielsweise sternförmig miteinander verbindet. Der Sensor-Hub kann ein Rechnernetz sein. Der Sensor-Hub kann als Kopplungselement mehrerer Sensoren bezeichnet werden. Der Sensor-Hub kann beispielsweise den Sensor am Pumpenkopf mit einem in der Sensornut des Motorgehäusemoduls aufgenommenen Sensor verbinden. Die Verbindung mehrerer Sensoren mittels eines Sensor-Hubs kann vorteilhaft sein, um eine Ausfallsicherheit gegenüber einem physikalischen Bus-Netz zu erhöhen. Der Sensor-Hub kann beispielsweise Kalibrier- und Identifikationsinformationen von der Pumpe und den Sensoren des Herzunterstützungssystems umfassen und über einen Kommunikationsbus im Anschlusskabel von einem zentralen Steuergerät des Herzunterstützungssystems auslesbar sein. Auf diese Weise kann das Steuergerät beispielsweise mit Motordaten parametriert werden. Der Sensor-Hub kann zudem dazu genutzt werden, Sensordaten der Sensoren der Pumpe vorzuverarbeiten, z. B. zu aggregieren, zu filtern, oder zu kalibrieren und das Kommunikationsprotokoll der Sensoren auf ein robusteres Kommunikationsprotokoll zu übersetzen und künstliche Redundanz oder Prüfsummen hinzuzufügen.

Vorteilhafterweise kann der Sensorleitungsabschnitt gemäß einer Ausführungsform einen Kontaktabschnitt aufweisen. Der Kontaktabschnitt kann an einer von dem Motorraum abgewandten Seite des Durchführungsabschnitts angeordnet sein. Zudem kann der Kontaktabschnitt O-förmig oder U-förmig ausgeformt sein. Vorteilhafterweise kann der Kontaktabschnitt zum elektrischen Kontaktieren der Sensorleitung mit dem Durchführungsabschnitt verwendet werden, wobei diese Ausführungsform besonders platzsparend ist. Der Kontaktabschnitt kann dazu beispielsweise als ein Endabschnitt des Sensorleitungsabschnitts ausgeformt sein und auf den Durchführungsabschnitt umgeklappt sein oder werden, wobei durch die O-Form oder U-Form beispielsweise das Kontaktieren des Anschlusskabels mit dem zumindest einen Kontaktstift ohne einen Kontakt des Kontaktabschnitts zur Durchführleitung realisiert werden kann.

Der Kontaktabschnitt kann gemäß einer Ausführungsform zumindest eine Kontaktfläche zum Anschließen an den zumindest einen Kontaktstift aufweisen. Die Kontaktfläche kann ausgeformt sein, um den zumindest einen Kontaktstift zumindest teilweise zu umschließen. Die Kontaktfläche kann dazu beispielsweise halbkreis- oder ellipsenförmig sein. Die Kontaktfläche kann beispielsweise einen offenliegenden elektrisch kontaktierbaren Bereich aufweisen, wobei der elektrische Kontakt zwischen dem Sensorleitungsabschnitt und dem Kontaktstift beispielsweise durch Lot oder Kleber hergestellt werden kann.

Gemäß einer Ausführungsform kann das Motorgehäusemodul eine Anschlussstellenkappe zum Abdecken einer Anschlussstelle zwischen dem Durchführungsabschnitt und dem Anschlusskabel aufweisen. Dies ist von Vorteil, um die Anschlussstelle zu schützen. Die Anschlussstellenkappe kann beispielsweise auch ein Teil der Sensorkappe sein. Die Anschlussstellenkappe kann, wie auch die Sensorkappe, mit einer Vergussmasse ausgefüllt werden, beispielsweise einem Silikon oder Epoxidharz, um Sensoren und Kontaktstellen vor Korrosion und leitfähigen Flüssigkeiten zu schützen. Die Anschlussstellenkappe kann flexibel ausgeformt sein, um neben einem mechanischen Schutz einen Knickschutz und eine Zugentlastung realisieren zu können.

Zudem kann das Motorgehäusemodul gemäß einer Ausführungsform eine Koppeleinrichtung zum Ankoppeln einer Einführeinrichtung zum Einführen des Herzunterstützungssystems an das Motorgehäusemodul aufweisen, insbesondere wobei die Koppeleinrichtung zumindest ein Fixierelement aufweisen kann. Dies ist von Vorteil, um das Motorgehäusemodul beispielsweise formschlüssig und/oder kraftschlüssig an die Einführeinrichtung fixieren zu können, beispielsweise um das Herzunterstützungssystem, welches das Motorgehäusemodul umfasst, minimalinvasiv einbringen zu können, und nach erfolgtem Implantieren der Einführeinrichtung abkoppeln zu können, um das Herzunterstützungssystem am Bestimmungsort freizusetzen. Das Fixierelement kann beispielsweise eine Klammer, auch Clamp genannt, oder dergleichen aufweisen. Die Koppeleinrichtung kann gemäß einer Ausführungsform auf dem Korpus des Motorgehäusemoduls realisiert sein.

Es wird zudem ein Herzunterstützungssystem vorgestellt. Das Herzunterstützungssystem weist ein Gehäuse mit einem Motorraum, einen Motor, der in dem Motorraum angeordnet ist, zumindest einen Sensor, eine mit dem zumindest einen Sensor elektrisch verbundene Sensorleitung, ein Anschlusskabel zum externen Kontaktieren des Herzunterstützungssystems und eine Ausführungsform des vorstehend genannten Motorgehäusemoduls als Teil des Gehäuses auf. Der Motor und der zumindest eine Sensor sind mittels des Motorgehäusemoduls elektrisch mit dem Anschlusskabel verbunden.

Bei dem Herzunterstützungssystem kann es sich um ein ventrikuläres Herzunterstützungssystem, insbesondere ein linksventrikuläres Herzunterstützungssystem handeln. Das Herzunterstützungssystem kann beispielsweise einen Elektromotor oder eine elektrisch betriebene Motor-Kupplung-Pumpeneinheit aufweisen. Der Sensor kann beispielsweise am Pumpenkopf und zusätzlich oder alternativ an dem Motorgehäusemodul angeordnet sein. Der Sensor kann beispielsweise ein Drucksensor sein oder ein Sensor zum Messen der Blutflussrichtung. Das Herzunterstützungssystem kann beispielsweise zum minimalinvasiven Einführen zylinderförmig sein und einen Durchmesser aufweisen, der geringer als die menschliche Aorta ist, beispielsweise 5 bis 12 Millimeter.

Zudem wird ein Verfahren zum Montieren eines Herzunterstützungssystems vorgestellt. Das Herzunterstützungssystem weist einen Motor, einen Motorraum, zumindest einen Sensor, eine mit dem zumindest einen Sensor elektrisch verbundene Sensorleitung und ein Anschlusskabel zum externen Kontaktieren des Herzunterstützungssystems auf. Das Verfahren umfasst einen Schritt des Bereitstellens, einen Schritt des Herstellens, einen Schritt des Kontaktierens und einen Schritt des Erzeugens. Im Schritt des Bereitstellens wird eine Ausführungsform des vorstehend genannten Motorgehäusemoduls bereitgestellt. Im Schritt des Herstellens wird eine elektrisch leitfähige Verbindung zwischen der zumindest einen Durchführleitung des Motorgehäusemoduls und dem Motor des Herzunterstützungssystems hergestellt. Im Schritt des Erzeugens wird eine stoffschlüssige Verbindung zwischen dem Motorgehäusemodul und dem Herzunterstützungssystem erzeugt, um den Motorraum des Herzunterstützungssystems abzudichten. Im Schritt des Kontaktierens wird der zumindest eine Kontaktstift des Motorgehäusemoduls mit der Sensorleitung des Herzunterstützungssystems kontaktiert.

Die stoffschlüssige Verbindung kann beispielsweise mittels Schweißen erzeugt werden. Optional kann nach dem Verschweißen zudem noch eine Sensorkappe und zusätzlich oder alternativ eine Anschlussstellenkappe zum Abdecken und Schützen eines elektronischen Bauteils oder einer elektrisch leitfähigen Schnittstelle einer Komponente des Herzunterstützungssystems montiert werden.

Das Verfahren kann gemäß einer Ausführungsform zudem einen Schritt des Anschließens des Anschlusskabels des Herzunterstützungssystems an die zumindest eine Durchführleitung und den zumindest einen Kontaktstift des Motorgehäusemoduls umfassen. Der Schritt des Anschließens kann vor oder nach dem Schritt des Erzeugens erfolgen. Wenn der Schritt des Anschließens nach dem Schritt des Erzeugens erfolgt, kann das Motorgehäusemodul eine Durchlassöffnung für das Anschlusskabel aufweisen.

Vorteilhafte Ausführungsbeispiele des hier vorgestellten Ansatzes sind in den Zeichnungen dargestellt und in der nachfolgenden Beschreibung näher erläutert. Es zeigt:
- Fig. 1: eine schematische Darstellung eines Herzunterstützungssystems gemäß einem Ausführungsbeispiel;
- Fig. 2: eine schematische Darstellung eines Motorgehäusemoduls gemäß einem Ausführungsbeispiel;
- Fig. 3: eine schematische Darstellung eines Motorgehäusemoduls gemäß einem Ausführungsbeispiel;
- Fig. 4: eine schematische Darstellung eines Motorgehäusemoduls gemäß einem Ausführungsbeispiel;
- Fig. 5: eine schematische Darstellung eines Sensorleitungsabschnitts eines Motorgehäusemoduls gemäß einem Ausführungsbeispiel;
- Fig. 6: eine schematische Darstellung eines Kappenelements für ein Motorgehäusemodul gemäß einem Ausführungsbeispiel;
- Fig. 7: eine schematische Darstellung eines Motorgehäusemoduls gemäß einem Ausführungsbeispiel;
- Fig. 8: eine schematische Darstellung eines Motorgehäusemoduls gemäß einem Ausführungsbeispiel; und
- Fig. 9: ein Ablaufdiagramm eines Verfahrens zum Montieren eines Herzunterstützungssystems gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

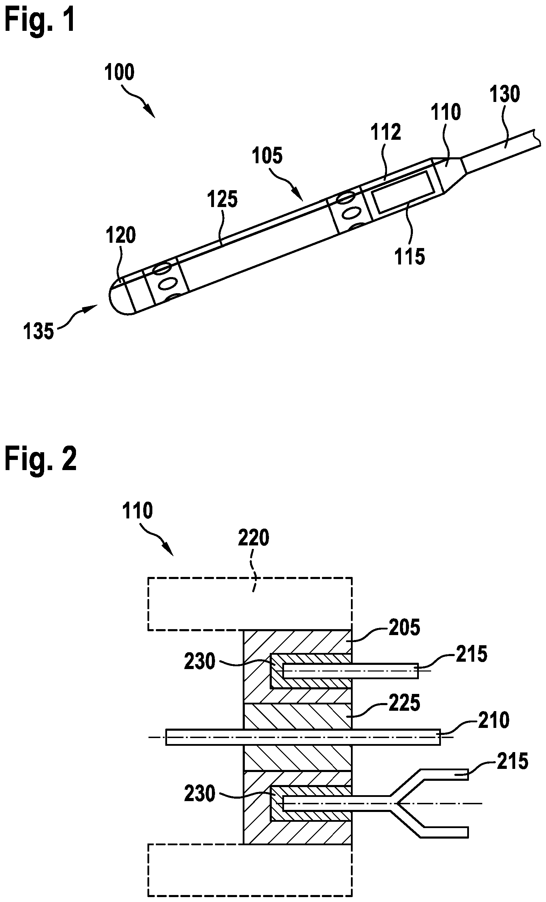

Fig. 1 zeigt eine schematische Darstellung eines Herzunterstützungssystems 100 gemäß einem Ausführungsbeispiel. Es ist eine Seitenansicht des Herzunterstützungssystems 100 gezeigt, das hier beispielhaft als linksventrikuläres Herzunterstützungssystems 100 ausgeführt ist. Das Herzunterstützungssystem 100 weist ein Gehäuse 105 auf. Als Teil des Gehäuses 105 umfasst das Herzunterstützungssystem 100 ein Motorgehäusemodul 110. Von dem Gehäuse 105 und dem Motorgehäusemodul 110 ist ein Motorraum 112 umschlossen. In dem Motorraum 112 ist ein Motor 115 angeordnet. An einer Kopfseite des Herzunterstützungssystems 100 ist in einer Sensorbaugruppe zumindest ein Sensor 120 angeordnet. Der Sensor 120 ist elektrisch mit einer Sensorleitung 125 verbunden. Die Sensorleitung 125 ist hier beispielhaft über das Gehäuse 105 zu dem Motorgehäusemodul 110 geführt, sie kann auch zumindest abschnittsweise innerhalb des Gehäuses 105 verlaufen oder spiralförmig über das Gehäuse 105 geführt sein. Der Sensor 120 kann beispielsweise ein Drucksensor oder ein Flusssensor zum Blutflussmessen sein, beispielsweise mittels Ultraschall oder Laser. An der dem Motorraum 112 abgewandten Seite des Motorgehäusemoduls 110 weist das Herzunterstützungssystem 100 ein Anschlusskabel 130 zum externen Kontaktieren des Herzunterstützungssystems 100 auf. Das Motorgehäusemodul 110 kann als elektrisches Verbindungselement bezeichnet werden: Der Motor 115 und der zumindest eine Sensor 120 sind mittels des Motorgehäusemoduls 110 elektrisch mit dem Anschlusskabel 130 verbunden. Das Motorgehäusemodul 110, auch Motor-Backend genannt, ist dazu ausgeformt, den Motorraum 112 hermetisch zu verschließen und somit fluiddicht abzudichten. Zudem ist das Motorgehäusemodul 110 dazu ausgebildet, eine elektrische Verbindung zwischen dem hermetisch verschlossenen Motorinneren des Motors 115 und der Umgebung des Herzunterstützungssystems 100 herzustellen: Das Motorgehäusemodul 110 übernimmt die Aufgaben der Zusammenführung der Sensorleitung 125, die elektrische Signale aus einem Pumpenkopf 135 des Herzunterstützungssystems 100 zum Motorgehäusemodul 110 führt, mit dem Anschlusskabel 130, das die Sensorsignale weiterführt und den Motor mit elektrischer Energie versorgt. Dazu können elektrische Leiter aus dem Inneren des Motors 115 mit dem auf der Außenseite des Motors 115 geführten Sensorkabel 125 und dem Anschlusskabel 130, auch Zuleitungskabel genannt, zusammengeführt werden. Auf diese Weise kann eine mechanisch sichere Verbindung des Anschlusskabels 130 mit dem Motorgehäusemodul 110 hergestellt werden. Über das Anschlusskabel 130 kann das Herzunterstützungssystem 100 an eine weitere Komponente angeschlossen sein oder werden, wie eine Energiequelle, eine Datenverarbeitungseinrichtung oder ein Steuergerät.

Das Herzunterstützungssystem 100 weist einen zylinderförmigen, länglichen Aufbau mit im Wesentlichen konstantem Außendurchmesser und abgerundeten, sich verjüngenden Enden zur einfachen Platzierung mittels eines Katheters in einem Blutgefäß, etwa der Aorta, auf. Das Motorgehäusemodul 110 hat die Form eines kegelstumpfs. Es ist konusförmig ausgeformt, mit einer Grundfläche in Richtung des Motorraums 112, die dem Außendurchmesser des Herzunterstützungssystems 100 entspricht, und mit einer kleineren Deckfläche als Übergang zum Anschlusskabel 130.

Fig. 2 zeigt eine schematische Darstellung eines Motorgehäusemoduls 110 zum Abdichten eines Motorraums eines Motors eines Herzunterstützungssystems gemäß einem Ausführungsbeispiel. Das Motorgehäusemodul 110 entspricht oder ähnelt hierbei dem Motorgehäusemodul 110 aus Fig. 1. Gezeigt ist ein Querschnitt einer Seitenansicht des Motorgehäusemoduls 110. Das Motorgehäusemodul 110 weist zumindest einen Durchführungsabschnitt 205 zum Herstellen einer elektrischen Verbindung zwischen dem Herzunterstützungssystem und einem Anschlusskabel zum externen Kontaktieren des Herzunterstützungssystems auf. Zudem weist das Motorgehäusemodul 110 zumindest eine Durchführleitung 210 auf, die in dem Durchführungsabschnitt 205 eingebettet ist und die sich durch den Durchführungsabschnitt 205 hindurch erstreckt. Die Durchführleitung 210 ist an den Motor und an das Anschlusskabel des Herzunterstützungssystems anschließbar. Das Motorgehäusemodul 110 weist ferner zumindest einen Kontaktstift 215 auf. Beispielhaft sind hier zwei unterschiedlich ausgeformte Kontaktstifte 215 gezeigt. Ein erstes Ende des Kontaktstifts 215 ist in dem Durchführungsabschnitt 205 eingebettet und ein zweites Ende ragt auf einer von dem Motorraum abgewandten Seite aus dem Durchführungsabschnitt 205 heraus. Das zweite Ende des Kontaktstifts 215 ist an eine Sensorleitung zu zumindest einem Sensor des Herzunterstützungssystems und an das Anschlusskabel anschließbar.

Der Durchführungsabschnitt 205 kann wie in dem hier gezeigten Ausführungsbeispiel zumindest eine mit einem elektrisch isolierenden Material verfüllte Durchgangsöffnung 225 zum Einbetten der zumindest einen Durchführleitung 210 und zumindest ein mit einem elektrisch isolierenden Material verfülltes Sackloch 230 zum Einbetten des zumindest einen Kontaktstifts 215 aufweisen. Eines der Sacklöcher kann auch leitfähig, beispielsweise mit elektrisch leitfähigem Kleber verfüllt sein, um eine elektrische Verbindung zwischen Motorgehäuse und einem Leiter des Anschlusskabels herzustellen. Dies kann beispielsweise der elektrischen Schirmung von Motor und Anschlusskabel dienen. Der Durchführungsabschnitt 205 ist beispielsweise aus Titan ausgeformt. Die Durchgangsöffnung 225 und die beiden gezeigten Sacklöcher 230 sind in dem Durchführungsabschnitt 205 ausgeformt und beispielsweise mit Glas als elektrisch isolierendes Material verfüllt. Die Sacklöcher 230 können entsprechend auch als blinde Glasdurchführungen bezeichnet werden, da sie nicht bis in das Innere des hermetisch verschlossenen Motors geführt sind. Die Durchführleitung 210, die als Durchführstift oder Pin realisiert sein kann, wird zur elektrischen Kontaktierung des Motors genutzt. Die Kontaktstifte 215, auch Blindpins genannt, werden zur Umverdrahtung der Sensorleitung genutzt. Die Durchführleitung 210 sowie der zumindest eine Kontaktstift 215 sind aus einem elektrisch leitfähigen Material ausgeformt, beispielsweise aus einem Metall wie einer Eisen-Nickel-Cobalt-Legierung mit einem geringen Wärmeausdehnungskoeffizienten oder wie Edelstahl.

Die zumindest eine Durchführleitung 210 und/oder der zumindest eine Kontaktstift 215 können zylinderförmig ausgeformt sein, wie hier beispielhaft bei der Durchführleitung 210 und dem oberen der beiden Kontaktstifte 215 gezeigt, also als gerade Pins. Die Durchführleitung 210 und/oder der zumindest eine Kontaktstift 215 können alternativ auch kelchförmig ausgeformt sein, wie beispielhaft bei dem unteren der beiden Kontaktstifte 215 gezeigt. Wenn die Durchführleitung 210 und/oder der zumindest eine Kontaktstift 215 zylinderförmig ausgeformt sind, kann die Anbindung des Anschlusskabels beispielsweise durch direktes Löten, Kleben, Crimpen oder Schweißen der Anschlusskabellitzen an die Durchführleitung 210 und/oder den Kontaktstift 215 oder unter Verwendung einer Hülse oder eines Steckers erfolgen. Wenn die Durchführleitung 210 und/oder der zumindest eine Kontaktstift 215 kelchförmig ausgeformt sind, kann die Kabelverbindung mit dem Anschlusskabel durch Einführen der Litzen in den Kelch realisiert werden. Eine Fixierung kann durch Löten, Kleben, Crimpen oder Schweißen erfolgen.

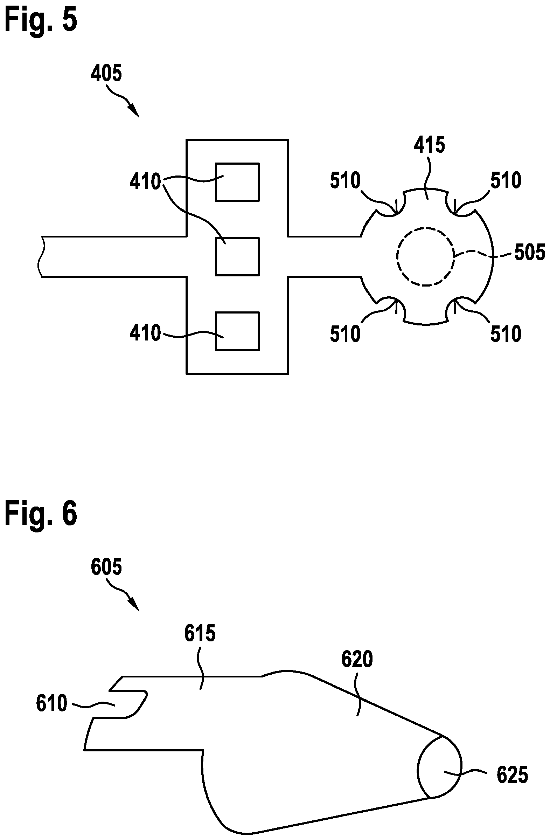

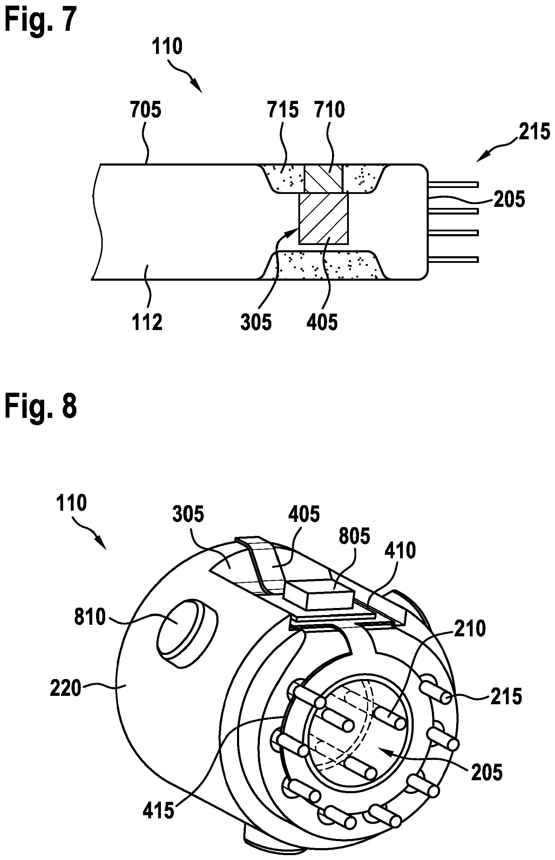

Das Motorgehäusemodul 110 ist gemäß dem in Fig. 2 dargestellten Ausführungsbeispiel zweiteilig ausgeführt, mit einem Korpus 220 und dem Durchführungsabschnitt 205, der beispielsweise als sogenannte Glas-Feedthrough-Komponente ausgeformt ist. Die zweiteilige Ausführung des Motorgehäusemoduls 110 ist produktionstechnisch vorteilhaft. Die elektrische Kontaktierung des Motors und der Sensorleitung mit dem Durchführungsabschnitt 205 kann in diesem Fall im Inneren des Motorgehäusemoduls 110, im Folgenden auch Backend genannt, ermöglicht werden, wobei Motorlitzen beispielsweise an den Durchführungsabschnitt 205 angelötet werden können. Von Vorteil bei der zweiteiligen Ausführung des Motorgehäusemoduls 110 ist, dass für den Durchführungsabschnitt 205 auf eine Standard-Glas-Durchführung, ein Standard-Glas-Feedthrough, zurückgegriffen werden kann, das dann beispielsweise durch Laserschweißen, Sintern oder Umspritzen hermetisch mit dem als Frästeil ausgeführten Korpus 220 kombiniert werden kann, wobei der Korpus weitere Merkmale, wie die Integration von Clamps als Fixierlement der Koppeleinrichtung und eine Sensormulde in Form der Sensornut aufweisen kann, wie beispielsweise anhand von Fig. 8 beschrieben. Die zweiteilige Ausführung des Motorgehäusemoduls 110 ist zudem in Bezug auf die Montage vorteilhaft, da beispielsweise folgender Produktionsablauf realisiert werden kann: Kontaktieren des Durchführungsabschnitts 205 mit dem Motorinneren, Verbinden des Durchführungsabschnitts 205 mit dem Korpus 220, beispielsweise durch Aufschieben des Korpus 220 über den Durchführungsabschnitt 205, Verschweißen des Korpus 220 mit dem Motorgehäuse 112, Verschweißen des Korpus 220 mit dem Durchführungsabschnitt 205, Herstellen einer elektrischen Verbindung des Sensorkabels an die Kontaktstifte 215 und Kontaktieren des Anschlusskabels mit der Durchführleitung 210 und den Kontaktstiften 215. Optional kann anschließend die Montage eines Kappenelements wie in Fig. 6 gezeigt als Schutzkappe mit Verguss-erfolgen.

Die zweiteilige Ausführung des Motorgehäusemoduls 110 kann durch eine Kombination aus einem Frästeil als Korpus 220 zur Herstellung der entsprechenden Geometrie mit vorteilhafter mechanischer Robustheit und Festigkeit und durch einen Durchführungsabschnitt 205 mit klassischen Glas-Durchführungen realisiert werden. Der Korpus 220 als Frästeil kann vorteilhafterweise aus Titan ausgeformt sein, um das Motorgehäusemodul 110 besonders einfach und effizient an ein Motorgehäuse 112 des Motors 115 anschweißen zu können, das beispielsweise auch aus Titan bestehen kann. Auf diese Weise kann eine hermetisch dichte Verbindung zwischen dem Korpus 220 und dem Motorgehäuse 112 hergestellt werden, um den Motorraum fluiddicht abzudichten. Die Ausformung der Kontaktstifte 215 als Glas-Blindstifte, also als blind endende Glasdurchführung, ermöglicht eine robuste Umverdrahtung der flexiblen Sensorleitung auf das Anschlusskabel auf Basis der Glas-Feedthrough-Technik, durch die Möglichkeit, die Kontaktstifte 215 an die Sensorleitung und an das Anschlusskabel anzuschließen. Fig. 2 zeigt somit ein Backend oder Motorgehäusemodul 110 mit Blind-Pins zur Umverdrahtung in Form der beiden beispielhaft gezeigten Kontaktstifte 215 in den Sacklöchern 230.

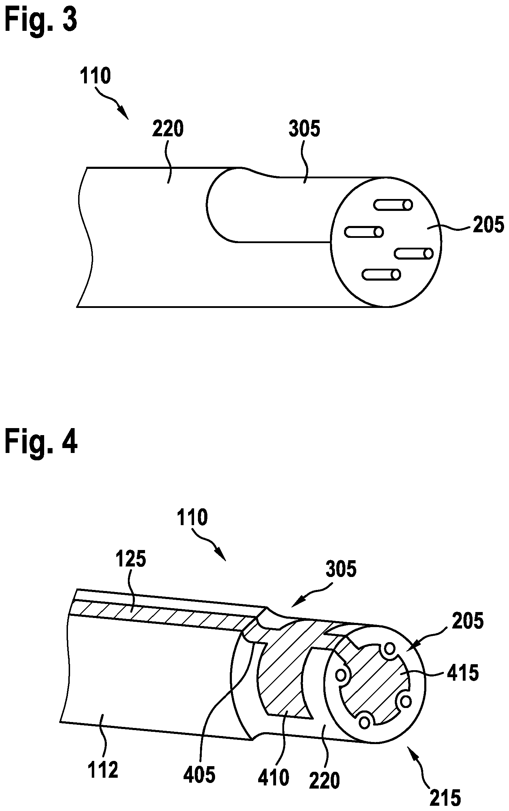

Fig. 3 zeigt eine schematische Darstellung eines Motorgehäusemoduls 110 gemäß einem Ausführungsbeispiel. Gezeigt ist eine Seitenansicht auf das Motorgehäusemodul 110 mit dem Korpus 220 und dem Durchführungsabschnitt 205, wobei der Durchführungsabschnitt 205 zum Einbetten der Durchführleitung und des zumindest einen Kontaktstifts ausgeformt ist und dazu beispielhafte Aussparungen aufweist.

Das Motorgehäusemodul 110, auch Pumpen-Backend genannt, weist eine zylinderförmige Form mit einer abgesetzten Ebene in Richtung des Durchführungsabschnitts 205 auf. Auf dieser abgesetzten Ebene kann beispielweise ein Sensor platziert werden. Die abgesetzte Ebene kann als Mulde oder als Kavität oder als Nut ausgeformt sein. Gemäß dem hier gezeigten Ausführungsbeispiel weist der Korpus 220 entsprechend eine Sensornut 305, in Form der abgesetzten Ebene, zum Aufnehmen zumindest eines elektronischen Bauteils, insbesondere eines Sensors und/oder eines Sensor-Hubs, auf.

In der Sensornut 305 kann ein elektrisch leitfähiges Substrat angeordnet werden, um eine elektrische Kontaktierung eines in der Sensornut 305 aufgenommenen elektronischen Bauteils zu realisieren. Das Substrat kann beispielsweise ausgeformt sein, um das in der Sensornut 305 aufgenommene elektronische Bauteil an elektrisch leitfähigen Pins des Backends, also an den zumindest einen in den Durchführungsabschnitt 205 eingebetteten Kontaktstift, anzuschließen. Das Substrat ist beispielsweise ein flexibles Dünnschichtsubstrat. Gemäß dem in der folgenden Fig. 4 gezeigten Ausführungsbeispiel kann das Substrat auch Teil der Sensorleitung oder eines Sensorleitungsabschnitts sein.

Optional kann das Motorgehäusemodul 110 eine Koppeleinrichtung zum Ankoppeln einer Einführeinrichtung an das Herzunterstützungssystem aufweisen, wie in Fig. 8 gezeigt. Zudem kann das Motorgehäusemodul 110 optional eine Passung zum Anbinden eines wie in Fig. 6 gezeigten Kappenelements als Schutzkappe oder als Knickschutztülle aufweisen. Das Kappenelement kann beispielweise ausgeformt sein, um die Sensornut 305 und den Durchführungsabschnitt 205 abzudecken.

Der Korpus 220 kann aus demselben Material wie der Motor des Herzunterstützungssystems ausgeformt sein, um eine hermetische Schweißverbindung zwischen dem Motor und dem Backend in Form des Motorgehäusemoduls 110 herstellen zu können. Eine feste Verbindung beispielsweise durch Ultraschallschweißen oder Anspritzen eines Polymers ist ebenfalls möglich, ebenso wie Sinterprozesse und Verglasungsprozesse von Keramikbauteilen, wenn das Motorgehäusemodul 110 beispielsweise Keramikkomponenten aufweist. Für die Verwendung des Motorgehäusemoduls 110 als elektrisches Verbindungselement ist der Durchführungsabschnitt 205 bedeutsam, der sowohl eine elektrische Durchführung in das hermetisch verschlossene Innere, als auch eine Umverdrahtung für die Sensorleitung realisieren kann. Eine Fertigung des Motorgehäusemoduls 110 aus einem Teil verzichtet auf eine Schweißnaht und erfordert entsprechend ausgeformte Glas-Durchführungen für den Durchführungsabschnitt 205.

Fig. 4 zeigt eine schematische Darstellung eines Motorgehäusemoduls 110 gemäß einem Ausführungsbeispiel. Es ist eine Seitenansicht des mit dem Motorraum 112 des Herzunterstützungssystems verbundenen Motorgehäusemoduls 110 gezeigt, wobei von dem Herzunterstützungssystem nur ein proximaler Abschnitt des zylinderförmigen Herzunterstützungssystems, das den Motorraum 112 umfasst, dargestellt ist. Das Motorgehäusemodul 110 weist auf der dem Motorraum 112 zugewandten Seite den gleichen Durchmesser und das gleiche Material auf, wie der Motorraum 112. Zur Ausbildung einer Sensornut kann sich das Motorgehäusemodul 110 konusförmig verjüngen, um Bauraum zur Platzierung von Sensoren zu schaffen. Die Sensorleitung 125 ist hier beispielhaft entlang der Längsachse des Herzunterstützungssystems 100 bandförmig auf dem Gehäuse des Herzunterstützungssystems über den Motorraum 112 zum Motorgehäusemodul 110 geführt.

Das Motorgehäusemodul 110 umfasst gemäß dem hier gezeigten Ausführungsbeispiel einen Sensorleitungsabschnitt 405 der Sensorleitung 125. Der Sensorleitungsabschnitt 405 weist im Bereich der Sensornut 305 einen Sensorträger 410 zum Anschließen des zumindest einen elektronischen Bauteils auf. Der Sensorträger 410 kann auch als ein Abschnitt, beispielsweise ein planarer Bereich des Motorgehäusemoduls 110 verstanden werden. Der Sensorleitungsabschnitt 405 ist beispielsweise zum Integrieren eines Sensors in der Sensornut 305 ausgeformt.

Die Sensorleitung 125 und der Sensorleitungsabschnitt 405 können aus einem elektrisch leitfähigen flexiblen Dünnschichtsubstrat ausgeformt sein. Die Sensornut 305 ist hier bandförmig um das Motorgehäusemodul 110 umlaufend ausgeformt. Der Sensorleitungsabschnitt 405 ist mit der Sensorleitung 125 verbunden, und verläuft zu einem Teil entlang der Sensornut 305 um einen Abschnitt der Mantelfläche des Motorgehäusemoduls 110, wobei der Sensorleitungsabschnitt 405 dazu in diesem Bereich ausgeweitet ist, um ein Ausformen mehrerer Sensorträger 410 zum Anschließen mehrerer elektronischer Bauteile auf dem Sensorleitungsabschnitt 405 entlang der Sensornut 305 zu ermöglichen, wie in der folgenden Fig. 5 gezeigt. Die Formgebung der Sensornut 305 kann entsprechend dem hier gezeigten Ausführungsbeispiel ausgeführt sein, um sowohl die Kabelführung der Sensorleitung 125 in dem beschriebenen Abschnitt des Sensorleitungsabschnitts 405 zu ermöglichen, als auch die Sensorintegration auf dem Sensorleitungsabschnitt 405 in der Sensornut 305 zu ermöglichen. Zu einem anderen Teil verläuft der Sensorleitungsabschnitt 405 in Richtung des Durchführungsabschnitts 205 von der Sensornut 305 aus auf die Querschnittsfläche des Durchführungsabschnitts 205.

Gemäß dem hier gezeigten Ausführungsbeispiel weist der Sensorleitungsabschnitt 405 einen Kontaktabschnitt 415 auf. Der Kontaktabschnitt 415 ist an einer von dem Motorraum 112 abgewandten Seite des Durchführungsabschnitts 205 angeordnet. Der Kontaktabschnitt 415 ist zumindest teilweise auf dem Durchführungsabschnitt 205 angeordnet. Der Kontaktabschnitt 415 kann O-förmig oder U-förmig ausgeformt sein. Hier erstreckt sich der Kontaktabschnitt 415 beispielhaft über einen Großteil der Querschnittsfläche des Durchführungsabschnitts 205. Der Kontaktabschnitt 415 weist gemäß dem hier gezeigten Ausführungsbeispiel im Bereich der Durchgangsöffnung und/oder der Sacklöcher Aussparungen auf. Zum Kontaktieren des zumindest einen Kontaktstifts 215 mit der Sensorleitung 125 kann der Sensorleitungsabschnitt 405 einen offenliegenden elektrisch kontaktierbaren Bereich in Form einer elektrisch leitfähigen Kontaktfläche 510 aufweisen, die an den in den Durchführungsbereich 205 eingebetteten zumindest einen Kontaktstifts 215 anschließt. Beispielhaft sind hier vier Kontaktstifte 215 gezeigt. Der Kontaktabschnitt 415 weist angrenzend an die Kontaktstifte je Kontaktstift 215 eine halbkreisförmige Aussparung 510 auf. Der Sensorleitungsabschnitt 405, und damit die Sensorleitung 125, ist über den Kontaktabschnitt 415 elektrisch mit den Kontaktstiften 215 im Druckführungsabschnitt 205 des Motormodulgehäuses 110 verbunden. Diese Ausführung der Verbindung kann auch als Anbindung der auch Sensor-Flex genannten flexiblen Sensorleitung 125 an die Blindpins in Form der Kontaktstifte 215 bezeichnet werden.

Fig. 5 zeigt eine schematische Darstellung eines Sensorleitungsabschnitts 405 eines Motorgehäusemoduls gemäß einem Ausführungsbeispiel. Der Sensorleitungsabschnitt 405 ist hier beispielhaft als Dünnschichtsubstrat zur Kontaktierung der Blindpins in Form der Kontaktstifte und zur Integration zusätzlicher Sensoren des Herzunterstützungssystems auf dem Motorgehäusemodul ausgeführt und in einer Aufsicht als Auffaltung gezeigt. Die hier gezeigte Ausformung des Sensorleitungsabschnitts 405 ist zur Kontaktierung der Sensorleitung an das Motorgehäusemodul geeignet und ermöglicht eine Sensorintegration auf dem Sensorleitungsabschnitt 405. Die Ausformung des Sensorleitungsabschnitts 405 entspricht im Wesentlichen dem in Fig. 4 beschriebenen Sensorleitungsabschnitt 405, mit der Ausweitung des Sensorleitungsabschnitts 405 in einen um das Motorgehäusemodul umlaufenden Anschnitt, der der Sensornut entspricht. In diesem Bereich der Ausweitung des Sensorleitungsabschnitts 405 sind in dem hier gezeigten Ausführungsbeispiel beispielhaft drei Sensorträger 410 ausgeformt. Auf diesen Sensorträgern 410 können elektronische Bauteile, beispielsweise Sensoren, integriert sein. Der Kontaktabschnitt 415 weist hier zusätzlich eine O-förmige Aussparung 505 auf, durch die die Durchführleitung durchgeführt werden kann, wenn der Kontaktabschnitt 415 auf dem Durchführungsabschnitt 205 aufliegt.

Der Kontaktabschnitt 415 weist zumindest eine Kontaktfläche 510 zum Anschließen an den zumindest einen Kontaktstift auf. Die zumindest eine Kontaktfläche 510 ist ausgeformt, um den zumindest einen Kontaktstift zumindest teilweise zu umschließen. Die Kontaktfläche 510 kann auch als Kontaktpad bezeichnet werden. Gemäß dem hier gezeigten Ausführungsbeispiel weist der Kontaktabschnitt 415 beispielhaft vier Kontaktflächen 510 auf, um vier in den Durchführungsabschnitt eingebettete Kontaktstifte elektrisch mit dem Sensorleitungsabschnitt 405 zu verbinden. Die Kontaktflächen 510 können je nach Ausformung des Kontaktabschnitts 415 halbkreisförmig oder ellipsenförmig ausgeformt sein, um je einen Kontaktstift zum elektrischen Kontaktieren mit dem Sensorleitungsabschnitt 405 zumindest teilweise zu umschließen. Die Form des Motorgehäusemoduls und der Sensorleitung sind beispielsweise durch die Ausformung des Sensorleitungsabschnitts 405 so aufeinander abgestimmt, dass die Kontaktpads 510 die Kontaktstifte des Motorgehäusemoduls umschließen. Die Kontaktpads 510 weisen dazu einen offenliegenden elektrisch kontaktierbaren Bereich auf. Ein elektrischer Kontakt kann beispielsweise durch Lot oder Kleber hergestellt werden. Die Kontaktierung der Durchführleitung zum Anschluss des Motors kann gleichartig wie die Kontaktierung der Kontaktstifte mit den Sensoren erfolgen, oder der Kontaktabschnitt 415 weist, wie hier gezeigt, die Aussparung 505 in O- oder U-Form auf, sodass eine Verbindung der Durchführleitung mit dem Anschlusskabel ohne Kontakt zum Kontaktabschnitt 415 des Sensorleitungsabschnitts 405 möglich ist. Die Anordnung des Kontaktabschnitts 415 auf dem Durchführungsabschnitt, und damit die Kontaktierung der Sensorleitung an die Blindpins des Motorgehäusemoduls, kann im Produktionsprozess beispielsweise durch ein Umklappen des Sensorleitungsabschnitts 405 auf den Durchführungsabschnitt und eine anschließende Herstellung der elektrischen Verbindungen erfolgen.

Durch zusätzliche Mulden in der Sensornut des Motorgehäusemoduls kann zusätzlicher Bauraum für die Aufnahme elektronischer Bauteile wie Sensoren in der Sensornut geschaffen werden, insbesondere wenn der Sensorleitungsabschnitt 405 wie hier gezeigt mehrere Sensorträger 410 aufweist. In der Sensornut aufgenommene Bauteile können zusätzlich durch ein Kappenelement mechanisch geschützt sein oder werden.

Ein auf dem Sensorleitungsabschnitt 405 in der Sensornut des Motorgehäusemoduls aufgenommenes elektronisches Bauteil kann gemäß einem Ausführungsbeispiel einen Sensor-Hub aufweisen. Der Sensor-Hub ist ausgebildet, zumindest ein Sensorsignal des zumindest einen Sensors des Herzunterstützungssystems zu verarbeiten. Zusätzlich oder alternativ ist der Sensor-Hub ausgebildet, das zumindest eine Sensorsignal über den zumindest einen Kontaktstift an das Anschlusskabel bereitzustellen. Die Integration eines Sensor-Hubs ermöglicht die Vorverarbeitung von Sensordaten sowie die Übersetzung der Datenschnittstellen. Darüber hinaus können Kalibrier- und Betriebsparameter wie eine Identifikationsinformation des Herzunterstützungssystems oder aufgenommener Sensoren mittels des Sensor-Hubs auf dem Herzunterstützungssystem gespeichert sein und mittels des Anschlusskabels an ein angeschlossenes Steuergerät bereitgestellt werden, beispielsweise über einen Kommunikationsbus im Anschlusskabel. Auf diese Weise kann das Steuergerät beispielsweise mit Motordaten parametriert werden. Der Sensor-Hub kann dazu genutzt werden, Sensordaten von Sensoren des Herzunterstützungssystems Pumpe vorzuverarbeiten, z. B. zu aggregieren, zu filtern, oder zu kalibrieren und das Kommunikationsprotokoll der Sensoren auf ein robusteres Kommunikationsprotokoll zu übersetzen (Transceiver) und künstliche Redundanz oder Prüfsummen hinzuzufügen.

Fig. 6 zeigt eine schematische Darstellung eines Kappenelements 605 für ein Motorgehäusemodul gemäß einem Ausführungsbeispiel. Das Kappenelement 605 ist zur Verwendung mit dem Motorgehäusemodul aus einer der hier gezeigten Figuren vorgesehen. Das Kappenelement 605 ist dazu ausgeformt, elektronische Bauteile eines Motorgehäusemoduls, wie es anhand von Fig. 3 beschrieben ist, abzudecken. Das Kappenelement 605 kann somit als mechanischer Schutz des Motorgehäusemoduls verwendet werden. Es ist eine Seitenansicht des Kappenelements 605 in einer einteiligen Ausführung gezeigt.

Das Kappenelement 605 weist in Richtung des Motorraums mindestens eine Aussparung 610 als sensitives Messfenster für einen der Sensoren 120/410/710 auf. Bei dem Sensor kann es sich beispielsweise um einen Drucksensor handeln, sodass das Messfenster 610 über der druckempfindlichen Membran des Drucksensors zu platzieren ist, sodass der Blutdruck des umgebenden Blutes ungehindert auf den Drucksensor einwirken kann. Angrenzend an die Aussparung 610 weist das Kappenelement die Sensorkappe 615 auf. Die Sensorkappe ist ausgeformt, um eine Sensornut, z. B. die in Fig. 3 beschriebene Sensornut, die beispielhaft als abgesetzte Ebene des zylinderförmigen Korpus des Motorgehäusemoduls ausgeformt ist, abzudecken. Wenn die Sensornut beispielsweise gemäß den in den Figuren 4 und 5 beschriebenen Ausführungsbeispielen als umlaufende Mulde ausgeformt ist, kann die Sensorkappe entsprechend zum Abdecken dieses Bereichs ausgeformt sein. An die Sensorkappe 615 schließt eine konusförmig in der Art einer Pfeilspitze ausgeformte Anschlussstellenkappe 620 an, die eine Öffnung 625 zum Durchführen des Anschlusskabels aufweist.

Das Kappenelement 605 weist gemäß dem hier gezeigten Ausführungsbeispiel somit die Sensorkappe 615 zum Abdecken des zumindest einen in der Sensornut aufgenommenen elektronischen Bauteils auf. Ferner weist das Kappenelement 605 die optionale Anschlussstellenkappe 620 zum Abdecken einer Anschlussstelle zwischen dem Durchführungsabschnitt und dem Anschlusskabel auf. Die Sensorkappe 615 und die Anschlussstellenkappe 620 können wie hier gezeigt als einteiliges Bauteil kombiniert als Kappenelement 605 ausgeführt sein.

Alternativ können die Sensorkappe 615 und die Anschlussstellenkappe 620 auch je als eigene Komponente getrennt ausgeführt sein. Die Sensorkappe 615 kann in diesem Fall beispielsweise eine metallische Kappe sein, die durch Kleben fixiert wird. Die Anschlussstellenkappe 620 kann beispielsweise flexibel ausgeformt sein, um neben einem mechanischen Schutz einen Knickschutz und eine Zugentlastung zu ermöglichen. Das Kappenelement 605 kann beispielsweise mit einer Vergussmasse ausgefüllt werden, beispielsweise mit einem Silikon oder Epoxidharz, um Sensoren und Kontaktstellen vor Korrosion und leitfähigen Flüssigkeiten zu schützen.

Fig. 7 zeigt eine schematische Darstellung eines Motorgehäusemoduls 110 gemäß einem Ausführungsbeispiel. Dabei entspricht oder ähnelt das Motorgehäusemodul 110 dem Motorgehäusemodul aus einer der vorstehend beschriebenen Figuren. Die Seitenansicht zeigt als Abschnitt des montierten zylinderförmigen Herzunterstützungssystems den Motorraum 112 mit einem Motorraumgehäuse 705. Das Motorgehäusemodul 110 ist mit dem Motorraumgehäuse 705 verbunden und weist in Richtung des Motorraums 112 eine umlaufende Mulde als Sensornut 305 auf. Im Bereich der Sensornut 305 ist auf dem Sensorleitungsabschnitt 405 als elektronisches Bauteil beispielhaft ein Sensor 710 integriert. Zur Veranschaulichung der Möglichkeit, den Sensor 710 unter Verwendung der Sensorkappe und/oder des Kappenelements mit einer Vergussmasse auszufüllen, wie anhand der vorhergehenden Fig. 6 beschrieben, ist der entsprechend ausgefüllte Bereich 715 hier beispielhaft gezeigt. Das Motorgehäusemodul 110 weist an der dem Motorraum 112 abgewandten Seite den Durchführungsabschnitt 205 auf, aus dem beispielhaft vier Kontaktstifte 215 herausragen.

Fig. 8 zeigt eine schematische Darstellung eines Motorgehäusemoduls 110 gemäß einem Ausführungsbeispiel. Das Motorgehäusemodul 110 ist hier in einer Aufsicht gezeigt. Der Korpus 220 ist als Titanteil realisiert. Zur elektrischen Funktionalisierung des Motorgehäusemoduls 110 als elektrisches Verbindungselement ist der Sensorleitungsabschnitt 405 aus der Richtung des Motorraums in die Sensornut 305 hineingeführt. Der Sensorleitungsabschnitt 405 ist hier beispielhaft als Dünnschichtsubstrat ausgeformt. Der Korpus 220 in Form eines Frästeils aus Titan weist als Sensornut 305 eine abgesetzte Ebene auf. Der Sensorleitungsabschnitt 405 weitet sich im Bereich der Sensornut 305 aus und füllt als dünne Schicht einen unteren Bereich der Grundfläche der Sensornut 305 fast vollständig aus. In der Sensornut 305 befindet sich auf dem Sensorleitungsabschnitt 405 ein Sensorträger 410, auf dem beispielhaft ein elektronisches Bauteil 805 aufgenommen ist.

Das Motorgehäusemodul 110 weist gemäß einem Ausführungsbeispiel eine Koppeleinrichtung zum Ankoppeln einer Einführeinrichtung zum Einführen des Herzunterstützungssystems an das Motorgehäusemodul 110 auf, insbesondere wobei die Koppeleinrichtung zumindest ein Fixierelement 810 aufweist. Das Fixierelement 810 kann der formschlüssigen Ankopplung eines Klammerelements, eines sogenannten Clamp, dienen. Der Korpus 220 als Titanteil weist hier beispielhaft drei runde Fixierelemente 810 als Koppeleinrichtung auf. Die Fixierelemente 810 können zusätzlich oder alternativ auch zum Fixieren eines Kappenelements zum Abdecken eines elektronischen Bauteils 805 oder einer elektrischen Anschlussstelle des Motorgehäusemoduls 110 verwendet werden, die Fixierelemente 810 dienen dann als Passung zum Anbringen des Kappenelements.

Das hier gezeigte Ausführungsbeispiel des Motorgehäusemoduls 110 weist den Korpus 220 und den Durchführungsabschnitt 205, realisiert als sogenanntes Glas-Feedthrough, auf. In dem Durchführungsabschnitt 205 sind beispielhaft drei Durchführleitungen 210 zum elektrischen Verbinden des Motors des Herzunterstützungssystems mit dem Anschlusskabel eingebettet. Zudem sind in dem Durchführungsabschnitt 205 beispielhaft acht U-förmig angeordnete Kontaktstifte 215 eingebettet. Die Kontaktstifte 215 sind im Wesentlichen gleichmäßig beabstandet angeordnet. Der Sensorleitungsabschnitt 405 ist bandförmig verjüngt in Richtung des Durchführungsabschnitts 205 aus der Sensornut 305 herausgeführt und bildet den O-förmigen Kontaktabschnitt 415 aus. Der Kontaktabschnitt 415 weist angrenzend an die Kontaktstifte 215 jeweils eine halbkreisförmige Kontaktfläche zum elektrischen Verbinden der Kontaktstifte 215 mit dem Sensorleitungsabschnitt 405 auf. Das Anschlusskabel kann zum externen Kontaktieren des Herzunterstützungssystems mittels des Motorgehäusemoduls 110 an die Durchführleitung 210 und an die Kontaktstifte 215 angeschlossen werden.

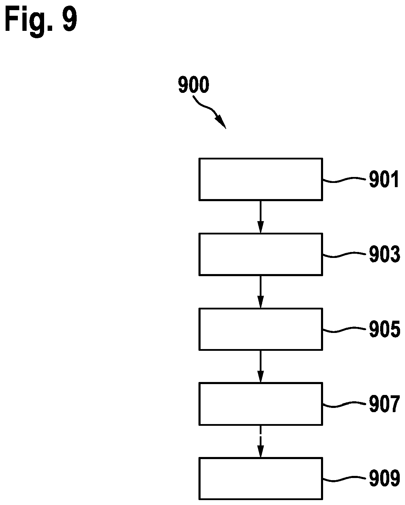

Fig. 9 zeigt ein Ablaufdiagramm eines Verfahrens 900 zum Montieren eines Herzunterstützungssystems gemäß einem Ausführungsbeispiel. Das Herzunterstützungssystem weist einen Motor, einen Motorraum, zumindest einen Sensor, eine mit dem zumindest einen Sensor elektrisch verbundene Sensorleitung und ein Anschlusskabel zum externen Kontaktieren des Herzunterstützungssystems auf. Das Verfahren 900 umfasst einen Schritt 901 des Bereitstellens, einen Schritt 903 des Herstellens, einen Schritt 905 des Kontaktierens und einen Schritt 907 des Erzeugens. Im Schritt 901 des Bereitstellens wird ein Motorgehäusemodul bereitgestellt. Dabei entspricht oder ähnelt das Motorgehäusemodul dem Motorgehäusemodul aus einer der vorstehend beschriebenen Figuren. Im Schritt 903 des Herstellens wird eine elektrisch leitfähige Verbindung zwischen der zumindest einen Durchführleitung des Motorgehäusemoduls und dem Motor des Herzunterstützungssystems hergestellt. Im Schritt 905 des Erzeugens wird eine stoffschlüssige Verbindung zwischen dem Motorgehäusemodul und dem Herzunterstützungssystem erzeugt, um den Motorraum des Herzunterstützungssystems abzudichten. Im Schritt 907 des Kontaktierens wird der zumindest eine Kontaktstift des Motorgehäusemoduls mit der Sensorleitung des Herzunterstützungssystems kontaktiert. Optional kann zudem im Schritt 907 des Erzeugens eine Sensorkappe und/oder eine Anschlussstellenkappe zum Abdecken und Schützen eines elektronischen Bauteils oder einer elektrisch leitfähigen Schnittstelle einer Komponente des Herzunterstützungssystems montiert werden.

Auch kann eine Reihenfolge der Schritte des hier vorgestellten Verfahrens in einem speziellen Ausführungsbeispiel wie folgt vorgesehen sein:
1. Durchführpin an Motorinneres anbinden
2. Korpus 220 aufführen
3. Korpus mit Motorgehäuse dicht verschweißen, damit die auf diese Weise hergestellte Verbindung mechanisch hält
4. Kontaktierelement in Korpus dicht verschweißen
5. Sensorleitung 125 aufkleben, Kontaktabschnitt 415 auf Durchführungsabschnitt 205 klappen, Kontaktfläche 510 an Kontaktstift 215 kontaktieren
6. Hülsen an Adern des Anschlusskabel 130 kontaktieren
7. Kontaktierte Hülsen auf Kontaktstift 215 und Durchführleitung 210 schieben und festschweißen
8. Verguss und Aufsetzen der Sensorkappe 615 und Anschlussstellenkappe 620

Das Verfahren 900 weist gemäß einem Ausführungsbeispiel optional einen Schritt 909 des Anschließens des Anschlusskabels des Herzunterstützungssystems an die zumindest eine Durchführleitung und den zumindest einen Kontaktstift des Motorgehäusemoduls auf. Der Schritt 909 des Anschließens ist vor oder nach dem Schritt 907 des Erzeugens ausführbar.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Motorgehäusemodul (110) zum Abdichten eines Motorraums (112) eines Motors (115) eines Herzunterstützungssystems (100),
mit einem Durchführungsabschnitt (205) zum Herstellen einer elektrischen Verbindung zwischen dem Herzunterstützungssystem (100) und einem Anschlusskabel (130) zum externen Kontaktieren des Herzunterstützungssystems (100);
mit zumindest einer Durchführleitung (210), die in dem Durchführungsabschnitt (205) eingebettet ist und sich durch den Durchführungsabschnitt (205) hindurch erstreckt, wobei die Durchführleitung (210) an den Motor (115) und an das Anschlusskabel (130) anschließbar ist;
**gekennzeichnet durch**
zumindest einen Kontaktstift (215), wobei ein erstes Ende des Kontaktstifts (215) in dem Durchführungsabschnitt (205) eingebettet ist und ein zweites Ende auf einer von dem Motorraum (112) abgewandten Seite aus dem Durchführungsabschnitt (205) herausragt, und wobei das zweite Ende des Kontaktstifts (215) an eine Sensorleitung (125) zu zumindest einem Sensor (120) des Herzunterstützungssystems (100) und an das Anschlusskabel (130) anschließbar ist.

2. Motorgehäusemodul (110) gemäß Anspruch 1, **dadurch gekennzeichnet, dass** der Durchführungsabschnitt (205) zumindest eine mit einem elektrisch isolierenden Material verfüllte Durchgangsöffnung (225) zum Einbetten der zumindest einen Durchführleitung (210) und zumindest ein mit einem elektrisch isolierenden Material verfülltes Sackloch (230) zum Einbetten des zumindest einen Kontaktstifts (215) aufweist, und/oder **dadurch gekennzeichnet, dass** die zumindest eine Durchführleitung (210) und/oder der zumindest eine Kontaktstift (215) zylinderförmig oder kelchförmig ausgeformt ist.

3. Motorgehäusemodul (110) gemäß einem der vorangegangenen Ansprüche, **gekennzeichnet durch** einen Korpus (220), wobei der Korpus (220) eine Sensornut (305) zum Aufnehmen zumindest eines elektronischen Bauteils (805), insbesondere eines Sensors (120) und/oder eines Sensor-Hubs, aufweist.

4. Motorgehäusemodul (110) gemäß Anspruch 3, **gekennzeichnet durch** eine Sensorkappe (615) zum Abdecken des zumindest einen in der Sensornut (305) aufgenommenen elektronischen Bauteils (805).

5. Motorgehäusemodul (110) gemäß Anspruch 3 oder 4, **gekennzeichnet durch** einen Sensorleitungsabschnitt (405) der Sensorleitung (125), wobei der Sensorleitungsabschnitt (405) im Bereich der Sensornut (305) einen Sensorträger (410) zum Anschließen des zumindest einen elektronischen Bauteils (805) aufweist.

6. Motorgehäusemodul (110) gemäß Anspruch 5, **dadurch gekennzeichnet, dass** das elektronische Bauteil (805) einen Sensor-Hub aufweist, und wobei der Sensor-Hub ausgebildet ist, zumindest ein Sensorsignal des zumindest einen Sensors (120) des Herzunterstützungssystems (100) zu verarbeiten und/oder über den zumindest einen Kontaktstift (215) an das Anschlusskabel (130) bereitzustellen.

7. Motorgehäusemodul (110) gemäß einem der Ansprüche 5 oder 6,
**dadurch gekennzeichnet, dass** der Sensorleitungsabschnitt (405) einen Kontaktabschnitt (415) aufweist, wobei der Kontaktabschnitt (415) an einer von dem Motorraum (112) abgewandten Seite des Durchführungsabschnitts (205) angeordnet ist.

8. Motorgehäusemodul nach Anspruch 7, **dadurch gekennzeichnet, dass** der Kontaktabschnitt (415) O-förmig oder U-förmig ausgeformt ist; und/oder **dadurch gekennzeichnet, dass** der Kontaktabschnitt (415) zumindest eine Kontaktfläche (510) zum Anschließen an den zumindest einen Kontaktstift (215) aufweist, und wobei die zumindest eine Kontaktfläche (510) ausgeformt ist, den zumindest einen Kontaktstift (215) zumindest teilweise zu umschließen.

9. Motorgehäusemodul (110) gemäß einem der vorangegangenen Ansprüche **gekennzeichnet durch** eine Anschlussstellenkappe (620) zum Abdecken einer Anschlussstelle zwischen dem Durchführungsabschnitt (205) und dem Anschlusskabel (130).

10. Motorgehäusemodul (110) gemäß einem der vorangegangenen Ansprüche, **gekennzeichnet durch** eine Koppeleinrichtung zum Ankoppeln einer Einführeinrichtung zum Einführen des Herzunterstützungssystems (100) an das Motorgehäusemodul (110).

11. Motorgehäusemodul (110) gemäß Anspruch 10, **dadurch gekennzeichnet, dass** die Koppeleinrichtung zumindest ein Fixierelement (810) aufweist.

12. Motorgehäusemodul nach einem der Ansprüche 1 bis 11, **dadurch gekennzeichnet, dass** das Herzunterstützungssystem (100) mittels Katheter in eine Herzkammer oder die Aorta einführbar ist.

13. Herzunterstützungssystem (100), wobei das Herzunterstützungssystem (100) folgende Merkmale aufweist:
ein Gehäuse (105) mit einem Motorraum (112);
einen Motor (115), der in dem Motorraum (112) angeordnet ist;
zumindest einen Sensor (120);
eine Sensorleitung (125), wobei die Sensorleitung (125) mit dem zumindest einen Sensor (120) elektrisch verbunden ist;
ein Anschlusskabel (130) zum externen Kontaktieren des Herzunterstützungssystems (100); und
ein Motorgehäusemodul (110) gemäß einem der vorangegangenen Ansprüche als Teil des Gehäuses (105), wobei der Motor (115) und der zumindest eine Sensor (120) mittels des Motorgehäusemoduls (110) elektrisch mit dem Anschlusskabel (130) verbunden sind.

14. Verfahren (900) zum Montieren eines Herzunterstützungssystems (100), wobei das Herzunterstützungssystem (100) einen Motor (115), einen Motorraum (112), zumindest einen Sensor (120), eine mit dem zumindest einen Sensor (120) elektrisch verbundene Sensorleitung (125) und ein Anschlusskabel (130) zum externen Kontaktieren des Herzunterstützungssystems (100) aufweist, wobei das Verfahren (900) folgende Schritte umfasst:
Bereitstellen (901) eines Motorgehäusemoduls (110) gemäß einem der Ansprüche 1 bis 12;
Herstellen (903) einer elektrisch leitfähigen Verbindung zwischen der zumindest einen Durchführleitung (210) des Motorgehäusemoduls (110) und dem Motor (115) des Herzunterstützungssystems (100);
Erzeugen (905) einer stoffschlüssigen Verbindung zwischen dem Motorgehäusemodul (110) und dem Herzunterstützungssystem (100), um den Motorraum (112) des Herzunterstützungssystems (100) abzudichten; und
Kontaktieren (907) des zumindest einen Kontaktstifts (215) des Motorgehäusemoduls (110) mit der Sensorleitung (125) des Herzunterstützungssystems (100).

15. Verfahren (900) gemäß Anspruch 14, mit einem Schritt (909) des Anschließen des Anschlusskabels (130) des Herzunterstützungssystems (100) an die zumindest eine Durchführleitung (210) und den zumindest einen Kontaktstift (215) des Motorgehäusemoduls (110).

## Claims

1. Motor housing module (110) for sealing a motor compartment (112) of a motor (115) of a cardiac assist system (100),
having a feedthrough portion (205) for establishing an electrical connection between the cardiac assist system (100) and a connection cable (130) for externally contacting the cardiac assist system (100);
having at least one feedthrough line (210), which is embedded in the feedthrough portion (205) and extends through the feedthrough portion (205), wherein the feedthrough line (210) can be connected to the motor (115) and to the connection cable (130);
**characterized by**
at least one contact pin (215), wherein a first end of the contact pin (215) is embedded in the feedthrough portion (205), and a second end protrudes out of the feedthrough portion (205) on a side facing away from the motor compartment (112), and wherein the second end of the contact pin (215) can be connected to a sensor line (125) to at least one sensor (120) of the cardiac assist system (100) and to the connection cable (130).

2. Motor housing module (110) according to claim 1, **characterized in that** the feedthrough portion (205) has at least one through-opening (225) filled with an electrically insulating material for embedding the at least one feedthrough line (210) and at least one blind hole (230) filled with an electrically insulating material for embedding the at least one contact pin (215), and/or
**characterized in that** the at least one feedthrough line (210) and/or the at least one contact pin (215) is cylindrical or cup-shaped.

3. Motor housing module (110) according to one of the preceding claims,
**characterized by** a body (220), wherein the body (220) has a sensor groove (305) for accommodating at least one electronic component (805), in particular a sensor (120) and/or a sensor hub.

4. Motor housing module (110) according to claim 3, **characterized by** a sensor cap (615) for covering the at least one electronic component (805) accommodated in the sensor groove (305).

5. Motor housing module (110) according to claim 3 or 4, **characterized by** a sensor line portion (405) of the sensor line (125), wherein the sensor line portion (405) has, in the region of the sensor groove (305), a sensor carrier (410) for connecting the at least one electronic component (805).

6. Motor housing module (110) according to claim 5, **characterized in that** the electronic component (805) has a sensor hub, and wherein the sensor hub is designed to process at least one sensor signal of the at least one sensor (120) of the cardiac assist system (100) and/or to provide it to the connection cable (130) via the at least one contact pin (215).

7. Motor housing module (110) according to one of claims 5 or 6,
**characterized in that** the sensor line portion (405) has a contact portion (415), wherein the contact portion (415) is arranged on a side of the feedthrough portion (205) that faces away from the motor compartment (112).

8. Motor housing module according to claim 7, **characterized in that** the contact portion (415) is O-shaped or U-shaped; and/or **characterized in that** the contact portion (415) has at least one contact surface (510) for connection to the at least one contact pin (215), and wherein the at least one contact surface (510) is shaped to enclose the at least one contact pin (215) at least partially.

9. Motor housing module (110) according to one of the preceding claims,
**characterized by** a connection point cap (620) for covering a connection point between the feedthrough portion (205) and the connection cable (130).

10. Motor housing module (110) according to one of the preceding claims,
**characterized by** a coupling device for coupling an insertion device for inserting the cardiac assist system (100) to the motor housing module (110).

11. Motor housing module (110) according to claim 10,
**characterized in that** the coupling device has at least one fixing element (810).

12. Motor housing module according to one of claims 1 to 11,
**characterized in that** the cardiac assist system (100) can be inserted into a cardiac ventricle or the aorta by means of a catheter.

13. Cardiac assist system (100), wherein the cardiac assist system (100) has the following features:
a housing (105) having a motor compartment (112);
a motor (115), which is arranged in the motor compartment (112);
at least one sensor (120);
a sensor line (125), wherein the sensor line (125) is electrically connected to the at least one sensor (120);
a connection cable (130) for externally contacting the cardiac assist system (100); and
a motor housing module (110) according to one of the preceding claims as part of the housing (105), wherein the motor (115) and the at least one sensor (120) are electrically connected to the connection cable (130) by means of the motor housing module (110).

14. Method (900) for assembling a cardiac assist system (100), wherein the cardiac assist system (100) has a motor (115), a motor compartment (112), at least one sensor (120), a sensor line (125) electrically connected to the at least one sensor (120), and a connection cable (130) for externally contacting the cardiac assist system (100), wherein the method (900) comprises the following steps:
providing (901) a motor housing module (110) according to one of claims 1 to 12;
establishing (903) an electrically conductive connection between the at least one feedthrough line (210) of the motor housing module (110) and the motor (115) of the cardiac assist system (100);
creating (905) an integrally bonded connection between the motor housing module (110) and the cardiac assist system (100) in order to seal the motor compartment (112) of the cardiac assist system (100); and
contacting (907) the at least one contact pin (215) of the motor housing module (110) with the sensor line (125) of the cardiac assist system (100).

15. Method (900) according to claim 14, comprising a step (909) of connecting the connection cable (130) of the cardiac assist system (100) to the at least one feedthrough line (210) and to the at least one contact pin (215) of the motor housing module (110).

## Revendications

1. Module de carter de moteur (110) destiné à étanchéifier un compartiment moteur (112) d'un moteur (115) d'un système d'assistance cardiaque (100),
comportant une section de passage (205) permettant d'établir une connexion électrique entre le système d'assistance cardiaque (100) et un câble de raccordement (130) destiné à la mise en contact externe du système d'assistance cardiaque (100) ;
comportant au moins une ligne de passage (210) intégrée dans la section de passage (205) et s'étendant à travers la section de passage (205), dans lequel la ligne de passage (210) peut être raccordée au moteur (115) et au câble de raccordement (130) ;
**caractérisé par**
au moins une broche de contact (215), dans lequel une première extrémité de la broche de contact (215) est intégrée dans la section de passage (205) et une seconde extrémité dépasse de la section de passage (205) sur un côté opposé au compartiment moteur (112), et dans lequel la seconde extrémité de la broche de contact (215) peut être raccordée à une ligne de capteur (125) vers au moins un capteur (120) du système d'assistance cardiaque (100) et au câble de raccordement (130).

2. Module de carter de moteur (110) selon la revendication 1,
**caractérisé en ce que** la section de passage (205) présente au moins une ouverture de passage (225) remplie d'un matériau électriquement isolant pour l'intégration de l'au moins une ligne de passage (210) et au moins un trou borgne (230) rempli d'un matériau électriquement isolant pour l'intégration de l'au moins une broche de contact (215), et/ou **caractérisé en ce que** l'au moins une ligne de passage (210) et/ou l'au moins une broche de contact (215) est moulée en forme de cylindre ou de calice.

3. Module de carter de moteur (110) selon l'une des revendications précédentes, **caractérisé par** un corps (220), dans lequel le corps (220) présente une rainure de capteur (305) destinée à recevoir au moins un composant électronique (805), en particulier un capteur (120) et/ou un concentrateur de capteur.

4. Module de carter de moteur (110) selon la revendication 3,
**caractérisé par** un bouchon de capteur (615) destiné à recouvrir l'au moins un composant électronique (805) reçu dans la rainure de capteur (305).

5. Module de carter de moteur (110) selon la revendication 3 ou 4,
**caractérisé par** une section de ligne de capteur (405) de la ligne de capteur (125), dans lequel la section de ligne de capteur (405) présente, dans la zone de la rainure de capteur (305), un support de capteur (410) permettant le raccordement de l'au moins un composant électronique (805).

6. Module de carter de moteur (110) selon la revendication 5,
**caractérisé en ce que** le composant électronique (805) présente un concentrateur de capteur, et dans lequel le concentrateur de capteur est conçu pour traiter au moins un signal de capteur de l'au moins un capteur (120) du système d'assistance cardiaque (100) et/ou pour le fournir au câble de raccordement (130) par l'intermédiaire de l'au moins une broche de contact (215).

7. Module de carter de moteur (110) selon l'une des revendications 5 ou 6,
**caractérisé en ce que** la section de ligne de capteur (405) présente une section de contact (415), dans lequel la section de contact (415) est disposée sur un côté de la section de passage (205) opposé au compartiment moteur (112).

8. Module de carter de moteur selon la revendication 7,
**caractérisé en ce que** la section de contact (415) est moulée en forme de O ou de U ; et/ou **caractérisé en ce que** la section de contact (415) présente au moins une surface de contact (510) destinée à être raccordée à l'au moins une broche de contact (215), et dans lequel l'au moins une surface de contact (510) est moulée de manière à entourer au moins partiellement l'au moins une broche de contact (215).

9. Module de carter de moteur (110) selon l'une des revendications précédentes, **caractérisé par** un bouchon de point de raccordement (620) destiné à recouvrir un point de raccordement entre la section de passage (205) et le câble de raccordement (130).

10. Module de carter de moteur (110) selon l'une des revendications précédentes, **caractérisé par** un dispositif d'accouplement destiné à l'accouplement d'un dispositif d'introduction permettant d'introduire le système d'assistance cardiaque (100) dans le module de carter de moteur (110).

11. Module de carter de moteur (110) selon la revendication 10,
**caractérisé en ce que** le dispositif d'accouplement présente au moins un élément de fixation (810).

12. Module de carter de moteur selon l'une des revendications 1 à 11,
**caractérisé en ce que** le système d'assistance cardiaque (100) peut être introduit dans un ventricule ou dans l'aorte à l'aide d'un cathéter.

13. Système d'assistance cardiaque (100), dans lequel le système d'assistance cardiaque (100) présente les caractéristiques suivantes :
un carter (105) comportant un compartiment moteur (112) ;
un moteur (115) disposé dans le compartiment moteur (112) ;
au moins un capteur (120) ;
une ligne de capteur (125), dans lequel la ligne de capteur (125) est connectée électriquement à l'au moins un capteur (120) ;
un câble de raccordement (130) destiné à la mise en contact externe du système d'assistance cardiaque (100) ; et
un module de carter de moteur (110) selon l'une des revendications précédentes en tant que partie du carter (105), dans lequel le moteur (115) et l'au moins un capteur (120) sont connectés électriquement au câble de raccordement (130) à l'aide du module de carter de moteur (110).

14. Procédé (900) permettant le montage d'un système d'assistance cardiaque (100), dans lequel le système d'assistance cardiaque (100) présente un moteur (115), un compartiment moteur (112), au moins un capteur (120), une ligne de capteur (125) connectée électriquement à l'au moins un capteur (120) et un câble de raccordement (130) destiné à la mise en contact externe du système d'assistance cardiaque (100), dans lequel le procédé (900) comprend les étapes suivantes :
fourniture (901) d'un module de carter de moteur (110) selon l'une des revendications 1 à 12 ;
établissement (903) d'une connexion électriquement conductrice entre l'au moins une ligne de passage (210) du module de carter de moteur (110) et le moteur (115) du système d'assistance cardiaque (100) ;
création (905) d'une liaison par coopération de matière entre le module de carter de moteur (110) et le système d'assistance cardiaque (100) afin de rendre étanche le compartiment moteur (112) du système d'assistance cardiaque (100) ; et
mise en contact (907) de l'au moins une broche de contact (215) du module de carter de moteur (110) avec la ligne de capteur (125) du système d'assistance cardiaque (100).

15. Procédé (900) selon la revendication 14, comportant une étape (909) de raccordement du câble de raccordement (130) du système d'assistance cardiaque (100) à l'au moins une ligne de passage (210) et à l'au moins une broche de contact (215) du module de carter de moteur (110).
